# EUROPÄISCHE PATENTSCHRIFT

(11) **EP 2 653 117 B1**
(45) Veröffentlichungstag und Bekanntmachung des Hinweises auf die Patenterteilung: **11.07.2018**
(21) Anmeldenummer: 13001767.6
(22) Anmeldetag: 06.04.2013
(51) Int. Cl.: A61B 17/29, A61B 17/00

(54) **Kupplung zwischen zwei Teilen eines medizinischen Instruments**
Coupling between two parts of a medical instrument
Accouplement entre deux parties d'un instrument médical

(30) Priorität: 18.04.2012 DE 102012007651
(43) Veröffentlichungstag der Anmeldung: 23.10.2013
(73) Patentinhaber: Karl Storz SE & Co. KG, 78532 Tuttlingen (DE)
(72) Erfinder: Stefan, Jochen, D-78532 Tuttlingen (DE)

(56) Entgegenhaltungen:
- EP-A2- 1 889 579
- DE-U1-202007 003 114
- US-A- 5 466 020
- US-A1- 2002 188 293
- US-A1- 2008 234 708
- US-A1- 2010 298 857
- US-A1- 2011 307 049

## Beschreibung

Die vorliegende Offenbarung ist auf ein Werkzeug, eine Handhabungseinrichtung und einen Schaft für ein medizinisches Instrument, ein medizinisches Instrument und ein Verfahren zum Herstellen einer Kupplung an einem proximalen Ende eines Werkzeugs oder an einem proximalen oder distalen Ende eines Schafts oder an einer Handhabungseinrichtung für ein medizinisches Instrument bezogen.

Die Vorschriften für die Reinigung und Sterilisierung wiederverwendbarer chirurgischer Instrumente stellen immer höhere Anforderungen, die eine immer weitergehende Zerlegbarkeit wiederverwendbarer chirurgischer Instrumente erfordert. Gleichzeitig werden insbesondere Instrumente für mikroinvasive Eingriffe immer kleiner, insbesondere die Querschnitte und Durchmesser von Schaft und Werkzeug am distalen Ende des Schafts. Kupplungen, die herkömmlich für die lösbare mechanische Kopplung von Werkzeug und Schaft vorgesehen sind, sind jedoch nicht beliebig miniaturisierbar. Bei einem Schaftdurchmesser von 3,5 mm oder weniger sind neue Konzepte zur lösbaren mechanischen Kopplung von Werkzeug und Schaft erforderlich.

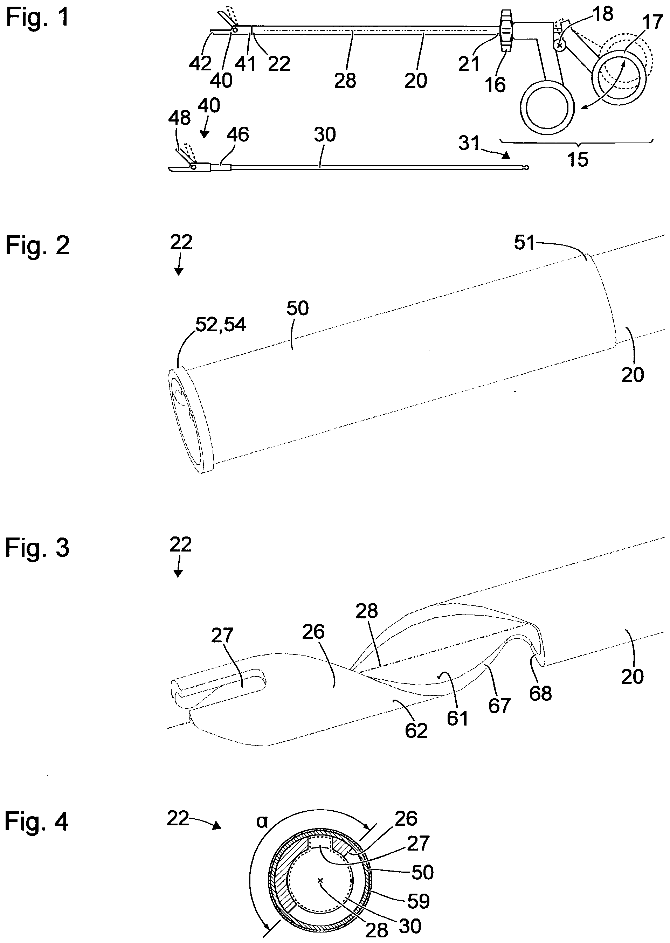

In EP 1 889 579 A2 ist ein medizinisches Rohrschaftinstrument beschrieben. Zur lösbaren Kopplung eines Werkzeugeinsatzes 15 mit einem schwenkbaren Maulteil 5b einerseits und eines hohlen Schafts 3 andererseits sind am Schaft 3 Steuerzapfen 23 und am Werkzeugeinsatz 15 entsprechende wendelförmige Führungsbahnen 22 vorgesehen (Absätze [0040], [0044] bis [0047], Figuren 4 bis 6c).

In US 2010/0298857 A1 ist eine Vorrichtung zur Zystektomie beschrieben. Zur lösbaren Verbindung einer Schneidspitze 40 mit einer Vorschubeinrichtung 90 sind in einer Rohrwand 92 an der Vorschubeinrichtung 90 ein spiralförmiger Schlitz 98 und an einem zylindrischen Hauptkörper 96 an der Schneidspitze 40 ein Bajonettfinger 106 zum Eingreifen in den spiralförmigen Schlitz 98 vorgesehen (Absätze [0073], [0074], Figur 8; ähnlich: Absätze [0095], [0105], [0106], Figuren 15, 16B).

In US 2008/0234708 A1 ist ein chirurgisches Instrument 900 mit einer zurückziehbaren Klammer 935 beschrieben (Absatz [0096], Figur 34 bis 37 und 42 bis 44). Für eine Trennbarkeit der Klammer 935 von dem chirurgischen Instrument sind an distalen und proximalen Abschnitten 938a, 938b eines rohrförmigen äußeren Bauglieds 938 und an distalen und proximalen Abschnitten 939a, 939b eines rohrförmigen inneren Bauglieds 939 jeweils korrespondierende Verbindungsbereiche zur Bildung von Bajonettverbindungen vorgesehen (Absatz [0099], Figuren 34, 42, 43, 44).

Eine Aufgabe der vorliegenden Erfindung besteht darin, ein verbessertes medizinisches Instrument zu schaffen.

Diese Aufgabe wird durch den Gegenstand des Anspruchs 1 gelöst. Weiterbildungen sind in den abhängigen Ansprüchen angegeben.

Ausführungsbeispiele der vorliegenden Erfindung beruhen auf der Idee, eine lösbare formschlüssige Verbindung zwischen Werkzeug und Schaft eines medizinischen Instruments durch zwei ineinander drehbare helikale Streifen zu schaffen. Die ineinandergreifenden helikal-streifenförmigen Kupplungsglieder an Schaft und Werkzeug bilden zusammen einen (insbesondere dünnwandigen) Kreiszylindermantel. Anders ausgedrückt kann man sich die beiden korrespondierenden Kupplungsglieder an Werkzeug und Schaft als durch einen Schnitt entlang eines geschlossenen Pfads aus einem kreiszylindermantelförmigen Rohr gebildet vorstellen, wobei der geschlossene Pfad (zumindest) zwei parallele helikale Abschnitte und zwischen deren Enden beispielsweise zwei in umfänglicher Richtung verlaufende Abschnitte aufweist.

Hinsichtlich der Relativbewegungen der beiden Kupplungsglieder beim Herstellen und beim Lösen einer mechanischen Verbindung besteht eine gewisse Ähnlichkeit mit einer Schraubverbindung. Die Kupplungsglieder bilden jedoch nicht ineinandergreifende Innen- und Außengewinde. Insbesondere ist weder ein hohler noch ein massiver Kern, an dem nach radial außen vorstehende Stege die Gewindegänge bilden (Außengewinde), erforderlich. Auch ein Mantel oder eine Hülse, an dem nach radial innen vorstehende Stege einen Gewindegang bilden (Innengewinde), ist nicht erforderlich. Vielmehr weisen die Kupplungsglieder jeweils näherungsweise die Gestalt eines Korkenziehers mit Seele auf. Die Kupplungsglieder sind also insbesondere - anders als die helikalen Stege an einem herkömmlichen Innen- oder Außengewinde - selbsttragend oder zumindest teilweise selbsttragend.

Ein einfacher Versuch mit zwei gleichen Korkenziehern mit Seele zeigt, dass der Kern eines herkömmlichen Außengewindes und der rohr-, hülsen- oder mantelförmige Bereich um ein herkömmliches Innengewinde, die bei einem Korkenzieher mit Seele fehlen, auch die Funktion haben, eine relative Verschiebung und eine relative Verkippung beider Gewinde zu verhindern. Die vorliegende Erfindung beruht auch auf der Idee, dass diese Funktion durch ein innerhalb oder außerhalb der Kupplungsglieder angeordnetes Element erfüllt werden kann, das mit keinem der beiden Kupplungsglieder starr verbunden ist. Vielmehr kann beispielsweise die Übertragungsstange zum Übertragen einer Kraft und/oder eines Drehmoments zwischen einem proximalen Ende und einem distalen Ende eines medizinischen Instruments so ausgebildet sein, dass sie im Wesentlichen innen an den Kupplungsgliedern anliegt. Obwohl die Übertragungsstange mit keinem der beiden Kupplungsglieder starr verbunden, sondern gegenüber beiden längs verschiebbar ist und/oder um die Längsachse rotierbar ist, kann sie eine relative Verschiebung senkrecht zur Längsachse und eine relative Verkippung der Kupplungsglieder verhindern, die andernfalls aufgrund unvermeidlicher Elastizität der Kupplungsglieder und aufgrund unvermeidlichen mechanischen Spiels zwischen den Kupplungsgliedern mögliche wäre.

Ausführungsbeispiele der vorliegenden Erfindung beruhen auf der Idee, eine lösbare formschlüssige Verbindung zwischen Werkzeug und Schaft eines medizinischen Instruments durch zwei ineinandergreifende Kupplungsglieder an Werkzeug und Schaft zu schaffen, wobei die Kupplungsglieder jeweils die Gestalt eines Ausschnitts eines Kreiszylindermantels aufweisen.

Ein Werkzeug für ein medizinisches Instrument umfasst ein Kupplungsglied am proximalen Ende des Werkzeugs, zur lösbaren mechanischen Kopplung mit einem distalen Endes eines Schafts des medizinischen Instruments, wobei das Kupplungsglied die Gestalt eines im Wesentlichen helikalen Ausschnitts eines Kreiszylindermantels aufweist.

Ein Werkzeug für ein medizinisches Instrument umfasst ein Kupplungsglied am proximalen Ende des Werkzeugs, zur lösbaren mechanischen Kopplung mit einem distalen Ende eines Schafts eines medizinischen Instruments, wobei das Kupplungsglied die Gestalt eines Ausschnitts eines Kreiszylindermantels aufweist, wobei das Werkzeug zur Kopplung mit einem Kupplungsglied, das ebenfalls die Gestalt eines Ausschnitts aus einem Kreiszylindermantel aufweist, am distalen Ende eines Schafts ausgebildet ist.

Insbesondere sind das Werkzeug und das Kupplungsglied zur Kopplung mit einem Kupplungsglied, das die Gestalt eines im Wesentlichen komplementären Ausschnitts des gleichen Kreiszylindermantels aufweist, vorgesehen und ausgebildet.

Das medizinische Instrument ist insbesondere ein Instrument für mikroinvasiv-chirurgische Eingriffe. Das medizinische Instrument umfasst insbesondere eine Handhabungseinrichtung mit einem oder mehreren Griffteilen, die manuell relativ zueinander bewegbar sein können, einen in der Regel langen und dünnen Schaft und das mittels des Kupplungsglieds mit dem distalen Ende des Schafts verbindbare Werkzeug. Das Werkzeug ist beispielsweise eine Zange, eine Schere, ein Stanzwerkzeug, ein Nadelhalter oder ein anderes Werkzeug mit einem oder zwei bewegbaren Maulteilen oder einem Haken oder einer anderen bewegbaren oder nicht bewegbaren Wirkeinrichtung.

Das Kupplungslied weist insbesondere insofern die Gestalt eines Ausschnitts eines Kreiszylindermantels mit einer vorbestimmten Wandstärke auf, als es eine innere und eine äußere Oberfläche aufweist, die durch einander entsprechende Ausschnitte aus der kreiszylindrischen inneren Mantelfläche des Kreiszylindermantels und aus der kreiszylindrischen äußeren Mantelfläche des Kreiszylindermantels gebildet werden. Anders als es beispielsweise bei einer herkömmlichen Bajonettkupplung die Knaggen, ragt kein Teil des Kupplungsglieds über die inneren Mantelfläche des Kreiszylindermantels nach innen oder über die äußere Mantelfläche des Kreiszylindermantels nach außen.

Bei einem Werkzeug, wie es hier beschrieben ist, weist das Kupplungsglied insbesondere die Gestalt eines helikal-streifenförmigen Ausschnitt eines Kreiszylindermantels oder die Gestalt eines Ausschnitts eines Kreiszylindermantels, dessen Breite nach proximal kontinuierlich oder diskontinuierlich abnimmt, auf.

Im Fall einer Gestalt eines helikal-streifenförmigen Ausschnitts eines Kreiszylindermantels weist das Kupplungsglied insbesondere eine konstante Breite auf, wobei die Breite jeweils in umfänglicher Richtung oder in Richtung senkrecht zu helikalen Kanten des Kupplungsglieds gemessen sein kann. Eine Streifenform bzw. eine konstante Breite kann eine besonders spielarme und präzise Kopplung mit einem entsprechend ausgebildeten Kupplungsglied eines Schafts ermöglichen.

Im Fall einer Gestalt eines Ausschnitts eines Kreiszylindermantels, dessen Breite nach proximal kontinuierlich abnimmt, ist die Breite insbesondere eine affin-lineare Funktion der parallel zur Längsachse von Werkzeug und Schaft gemessenen Koordinate. Diese Gestalt wird auch als helikal-keilförmige Gestalt bezeichnet. Mit einer nach proximal kontinuierlich oder diskontinuierlich bzw. stufenförmig abnehmenden Breite ist eine Anpassung der Breite an die an jedem Ort aufzunehmenden oder zu übertragenden Kräfte und Momente möglich. Das Kupplungsglied kann an seinem distalen Ende, an dem es die größten Kräfte und Momente übertragen muss, breit, und an seinem proximalen Ende schmal ausgebildet sein. Umgekehrt kann ein korrespondierendes Kupplungsglied am distalen Ende eines Schafts distal schmal und proximal breit ausgebildet sein. Dies kann eine insgesamt hohe Steifheit der Kupplung von Werkzeug und Schaft ermöglichen. Ferner kann eine nach proximal abnehmende Breite eine Kopplung mit einem Kupplungsglied, dessen Breite nach distal abnimmt, vereinfachen.

Das Kupplungsglied kann verhältnismäßig dünnwandig ausgebildet sein, insbesondere dünnwandiger als eine Gewindehülse mit einem Innen- oder Außengewinde. Dadurch kann das Kupplungsglied eine weitergehende Miniaturisierung ermöglichen.

Bei einem Werkzeug, wie es hier beschrieben ist, ist das Kupplungsglied insbesondere aus einem Rohr gebildet.

Das Rohr, aus dem das Kupplungsglied gebildet ist, weist insbesondere einen kreisringförmigen Querschnitt auf. Das Kupplungsglied ist insbesondere mittels Laserschneidens aus dem Rohr gebildet. Da dabei keine Kraft auf das Rohr wirkt und es deshalb nicht verformt wird, kann das Kupplungsglied durch Laserschneiden besonders präzise gefertigt werden.

Ein Werkzeug, wie es hier beschrieben ist, umfasst insbesondere ferner eine Übertragungsstange zum Übertragen zumindest entweder einer Kraft oder eines Drehmoments zwischen einem proximalen Ende und einem distalen Ende eines mit dem Werkzeug gekoppelten Schafts, wobei der Innendurchmesser des Kreiszylindermantels im Wesentlichen dem Außendurchmesser der Übertragungsstange entspricht.

Der Innendurchmesser des Kreiszylindermantels entspricht insbesondere bis auf das für eine reibungsarme Bewegbarkeit der Übertragungsstange erforderliche Spiel dem Außendurchmesser der Übertragungsstange. Die Übertragungsstange weist dazu insbesondere einen kreisförmigen Querschnitt auf. Alternativ weist die Übertragungsstange im Bereich des Kupplungsglieds beispielsweise einen sternförmigen oder polygonalen Querschnitt auf, so dass sie entlang mehrerer Linien innen am Kupplungsglied anliegt oder im Wesentlichen anliegt und dieses stützen kann. Das Werkzeug und die Übertragungsstange können so ausgebildet und miteinander mechanisch gekoppelt sein, dass eine Bewegung der Übertragungsstange relativ zum Werkzeug nach proximal eine schließende und nach distal eine öffnende Bewegung am Werkzeug (oder umgekehrt) bewirkt.

Wie bereits erwähnt, kann die Übertragungsstange, obwohl sie mit dem Kupplungsglied nicht starr verbunden, sondern relativ zu diesem bewegbar ist, das Kupplungsglied von innen stützen und eine erwünschte Positionierung und Ausrichtung des Kupplungsglieds des Werkzeugs relativ zu einem korrespondierenden Kupplungsglied am distalen Ende eines Schafts erzwingen. Diese doppelte Funktion bzw. Aufgabe der Übertragungsstange kann zur Miniaturisierbarkeit des Kupplungsglieds beitragen.

Ein Werkzeug, wie es hier beschrieben ist, umfasst insbesondere ferner eine Stützhülse, die an dem Kupplungsglied anliegt und mit dem Kupplungsglied gefügt ist.

Die Stützhülse kann innen oder außen am Kupplungsglied anliegen und mit diesem insbesondere durch Laserschweißen oder Löten verbunden sein. Das Kupplungsglied kann - wie bereits erwähnt - in erheblichem Umfang nicht nur zur Aufnahme, sondern (anders als ein helikaler Steg an einem herkömmlichen Gewinde) auch für eine weiträumige Ableitung bzw. Übertragung von Kräften ausgebildet sein. Deshalb kann die Stützhülse besonders dünnwandig ausgebildet sein, insbesondere dünnwandiger als eine Hülse, an der durch Gewindeschneiden ein herkömmliches Gewinde erzeugt ist. Darüber hinaus können das Fertigen des Kupplungsglieds und das Fügen der dünnwandigen Stützhülse an das Kupplungsglied mit geringerem Material- und Zeitaufwand und bei geringerem Ausschuss durchführbar sein als die Fertigung eines herkömmlichen Gewindes durch Gewindeschneiden.

Bei einem Werkzeug, wie es hier beschrieben ist, weist der helikal-streifenförmige Ausschnitt des Kreiszylindermantels insbesondere in umfänglicher Richtung eine Breite entsprechend dem halben Umfang des Kreiszylindermantels auf.

Die umfängliche Richtung bezieht sich auf den Kreiszylindermantel bzw. dessen Umfang (in einer Ebene senkrecht zur Symmetrieachse des Kreiszylindermantels). Ein Kupplungsglied mit der genannten Breite korrespondiert zu und ist koppelbar mit einem Kupplungsglied der gleichen Breite. Dabei ist mit einer Breite entsprechend dem halben Umfang des Kreiszylindermantels auch eine Breite gemeint, die geringfügig kleiner als der halbe Umfang ist, um durch ein kleines Spiel eine reibungsarme Koppelbarkeit der Kupplungsglieder zu ermöglichen. Wenn beide Kupplungsglieder des Werkzeugs und des Schafts im Wesentlichen die gleiche Breite aufweisen, ermöglicht dies bei gegebener Wandstärke der Kupplungsglieder eine maximale Steifigkeit der Kupplung des Werkzeugs und des Schafts.

Alternativ kann der helikal-streifenförmige Ausschnitt des Kreiszylindermantels in umfänglicher Richtung eine Breite aufweisen, die kleiner ist als der halbe Umfang des Kreiszylindermantels, insbesondere eine Breite im Bereich von einem Viertel bis zu einem Drittel des Umfangs des Kreiszylindermantels. Dies gilt insbesondere, wenn eine Stützhülse vorgesehen ist. Die versteifende Wirkung der Stützhülse auf das Kupplungsglied am Werkzeug kann also genutzt werden, um das Kupplungsglied am Werkzeug schmäler und das korrespondierende Kupplungsglied am Schaft breiter auszubilden und so die Gesamtsteifigkeit des Kupplungsglieds am Schaft zu erhöhen.

Ein Werkzeug, wie es hier beschrieben ist, umfasst insbesondere ferner einen Riegel, der relativ zum Kupplungsglied axial verschiebbar ist.

Mit einer axialen Verschiebbarkeit des Kupplungsglieds ist eine Verschiebbarkeit parallel zur Längs- und/oder Symmetrieachse von Schaft, Übertragungsstange und Kreiszylindermantel gemeint. Der Riegel ist insbesondere dazu ausgebildet, in eine korrespondierende Ausnehmung am Kupplungsglied eines mit dem Werkzeug zu koppelnden Schafts einzugreifen, um die mechanische Kopplung von Werkzeug und Schaft zu verriegeln.

Ein Schaft für ein medizinisches Instrument umfasst ein Kupplungsglied am distalen Ende des Schafts zur lösbaren mechanischen Kopplung mit einem proximalen Ende eines Werkzeugs zur Bildung eines medizinischen Instruments, wobei das Kupplungsglied die Gestalt eines im Wesentlichen helikalen Ausschnitts eines Kreiszylindermantels aufweist.

Ein Schaft für ein medizinisches Instrument umfasst ein Kupplungsglied am distalen Ende des Schafts, zur lösbaren mechanischen Kopplung mit einem proximalen Ende eines Werkzeugs, wobei das Kupplungsglied die Gestalt eines helikalen Ausschnitts eines Kreiszylindermantels aufweist.

Das Werkzeug ist insbesondere zur Kopplung mit einem Kupplungsglied, das ebenfalls die Gestalt eines Ausschnitts aus einem Kreiszylindermantel aufweist, am proximalen Ende eines Werkzeugs ausgebildet.

Insbesondere sind der Schaft und das Kupplungsglied zur Kopplung mit einem Kupplungsglied, das die Gestalt eines im Wesentlichen komplementären Ausschnitts des gleichen Kreiszylindermantels aufweist, vorgesehen und ausgebildet.

Bei einem Schaft, wie er hier beschrieben ist, ist das Kupplungsglied insbesondere aus einem Rohr gebildet.

Bei einem Schaft, wie er hier beschrieben ist, weist das Kupplungsglied insbesondere die Gestalt eines helikal-streifenförmigen Ausschnitt eines Kreiszylindermantels oder die Gestalt eines Ausschnitts eines Kreiszylindermantels, dessen Breite nach distal kontinuierlich oder diskontinuierlich abnimmt, auf.

Bei einem Schaft, wie er hier beschrieben ist, entspricht insbesondere der Innendurchmesser des Kreiszylindermantels im Wesentlichen dem Außendurchmesser einer Übertragungsstange, für die der Schaft ausgebildet ist.

Ein Schaft, wie er hier beschrieben ist, umfasst insbesondere ferner eine Stützhülse, die an dem Kupplungsglied anliegt und mit dem Kupplungsglied gefügt ist.

Die Stützhülse liegt insbesondere außen an dem Kupplungsglied an.

Bei einem Schaft, wie er hier beschrieben ist, weist insbesondere der helikal-streifenförmige Ausschnitt des Kreiszylindermantels in umfänglicher Richtung eine Breite entsprechend dem halben Umfang des Kreiszylindermantels auf.

Alternativ weist bei einem Schaft, wie er hier beschrieben ist, der helikal-streifenförmige Ausschnitt des Kreiszylindermantels in umfänglicher Richtung eine Breite auf, die kleiner als der halbe Umfang des Kreiszylindermantels ist. Dies gilt insbesondere, wenn die oben beschriebene Stützhülse vorgesehen ist.

Eine Kupplung für ein Teil eines zerstörungsfrei zerlegbaren medizinischen Instruments oder an einem Teil eines zerstörungsfrei zerlegbaren medizinischen Instruments umfasst ein Kupplungsglied zur lösbaren mechanischen Kopplung mit einem korrespondierenden Kupplungsglied an einem anderen Teil des medizinischen Instruments, wobei das Kupplungsglied die Gestalt eines im Wesentlichen helikalen Ausschnitts eines Kreiszylindermantels aufweist.

Ein zerstörungsfrei zerlegbares medizinisches Werkzeug ist ein medizinisches Werkzeug, das - insbesondere ohne Verwendung eines Schraubendrehers, eines Schraubenschlüssels oder irgendeines anderen Werkzeugs - wiederholt in zwei oder mehr Teile zerlegt und anschließend wieder vollständig und funktionsfähig zusammengesetzt werden kann. Die Zerlegbarkeit ermöglicht oder vereinfacht insbesondere die Reinigung und Sterilisierung des medizinischen Instruments und/oder den Austausch eines Teils des medizinischen Instruments.

Die Kupplung ist insbesondere an einem distalen Ende eines Schafts oder an einem proximalen Ende eines Schafts oder an einem distalen Ende einer Handhabungseinrichtung vorgesehen und angeordnet. Alternativ ist die Kupplung beispielsweise für ein distales Ende eines Schafts oder für ein proximales Ende eines Schafts oder für ein distales Ende einer Handhabungseinrichtung vorgesehen.

Insbesondere ist das Kupplungsglied zur Kopplung mit einem korrespondierenden Kupplungsglied, das die Gestalt eines im Wesentlichen komplementären Ausschnitts des gleichen Kreiszylindermantels aufweist, vorgesehen und ausgebildet.

Bei einer Kupplung, wie sie hier beschrieben ist, ist das Kupplungsglied insbesondere aus einem Rohr gebildet.

Bei einer Kupplung, wie sie hier beschrieben ist, weist das Kupplungsglied insbesondere die Gestalt eines helikal-streifenförmigen Ausschnitt eines Kreiszylindermantels oder die Gestalt eines Ausschnitts eines Kreiszylindermantels, dessen Breite nach distal kontinuierlich oder diskontinuierlich abnimmt, auf.

Bei einer Kupplung, wie sie hier beschrieben ist, entspricht insbesondere der Innendurchmesser des Kreiszylindermantels im Wesentlichen dem Außendurchmesser einer Übertragungsstange eines medizinischen Instruments, für das die Kupplung ausgebildet ist.

Eine Kupplung, wie sie hier beschrieben ist, umfasst insbesondere ferner eine Stützhülse, die an dem Kupplungsglied anliegt und mit dem Kupplungsglied gefügt ist.

Die Stützhülse liegt insbesondere außen an dem Kupplungsglied an.

Bei einer Kupplung, wie sie hier beschrieben ist, weist insbesondere der helikal-streifenförmige Ausschnitt des Kreiszylindermantels in umfänglicher Richtung eine Breite entsprechend dem halben Umfang des Kreiszylindermantels auf.

Alternativ weist bei einer Kupplung, wie sie hier beschrieben ist, der helikal-streifenförmige Ausschnitt des Kreiszylindermantels in umfänglicher Richtung eine Breite auf, die kleiner als der halbe Umfang des Kreiszylindermantels ist. Dies gilt insbesondere, wenn die oben beschriebene Stützhülse vorgesehen ist.

Eine Handhabungseinrichtung oder ein Schaft für ein zerstörungsfrei zerlegbares medizinisches Instrument umfasst eine Kupplung, wie sie hier beschrieben ist.

Bei einer Handhabungseinrichtung mit der Kupplung ist die Kupplung insbesondere am distalen Ende der Handhabungseinrichtung angeordnet. Bei einem Schaft mit der Kupplung ist die Kupplung insbesondere am distalen Ende oder am proximalen Ende des Schafts angeordnet.

Bei einer Kupplung, wie sie hier beschrieben ist, schließen die Ränder des helikal-streifenförmigen Ausschnitts mit der Symmetrieachse des Kreiszylindermantels insbesondere einen Winkel im Bereich von 40° bis 70° ein.

Insbesondere liegt der Winkel im Bereich von 50° bis 65°. Der Winkel beeinflusst bei einer vorgegebenen Anzahl von Windungen die Länge des Kupplungsglieds. Je geringer die Steigung bzw. je größer der Winkel ist, desto kürzer ist das Kupplungsglied. Gleichzeitig beeinflusst der Winkel die Breite des helikal-streifenförmigen Ausschnitts, gemessen in einer Richtung senkrecht zu dessen helikalen Rändern. Diese Breite gemessen senkrecht zu den helikalen Rändern des Kupplungsglieds hat Einfluss auf die Steifigkeit des Kupplungsglieds. Je größer die Steigung bzw. je kleiner der Winkel ist, desto größer sind die Breite des Kupplungsglieds in Richtung senkrecht zu dessen helikalen Rändern und seine Steifigkeit im Verhältnis zu seiner Länge. Ein Winkel im Bereich von 40° bis 70° und mehr noch ein Winkel im Bereich von 50° bis 65° bietet einen besonders guten Kompromiss und damit insgesamt eine besonders hohe Steifigkeit des Kupplungsglieds.

Weitere mögliche Ausführungsformen, Varianten, Eigenschaften, Merkmale, Wirkungen und Vorteile einer Kupplung entsprechen den oben für ein Werkzeug beschriebenen.

Ein medizinisches Instrument umfasst ein Werkzeug, wie es hier beschrieben ist, und einen Schaft, wie er hier beschrieben ist.

Ein medizinisches Instrument umfasst einen Schaft, ein Werkzeug, ein erstes Kupplungsglied am distalen Ende des Schafts und ein zweites Kupplungsglied am proximalen Ende des Werkzeugs, zur lösbaren mechanischen Kopplung mit dem ersten Kupplungsglied, wobei das erste Kupplungsglied die Gestalt eines Ausschnitts aus einem ersten Kreiszylindermantel und das zweite Kupplungsglied die Gestalt eines Ausschnitts aus einem zweiten Kreiszylindermantel aufweisen, wobei der erste Kreiszylindermantel und der zweite Kreiszylindermantel die gleichen Durchmesser aufweisen.

Bei einem medizinischen Instrument, wie es hier beschrieben ist, sind insbesondere die Kupplungsglieder an Schaft und Werkzeug gleich.

Eine Gleichheit der Kupplungsglieder bezieht sich insbesondere auf die miteinander korrespondierenden Eigenschaften der Kupplungsglieder und schließt nicht aus, dass die die Kupplungsglieder bildenden Bauteile sich beispielsweise in der Art der mechanischen Verbindung mit Schaft bzw. Werkzeug unterscheiden.

Ein medizinisches Instrument, wie es hier beschrieben ist, umfasst ferner insbesondere am proximalen Ende eine Handhabungseinrichtung zur Handhabung des medizinischen Instruments, wobei die Handhabungseinrichtung mit dem proximalen Ende des Schafts verbunden oder lösbar verbindbar ist.

Bei einem Verfahren zum Herstellen einer Kupplung für oder an einem proximalen Ende eines Werkzeugs für ein medizinisches Instrument oder für oder an einem distalen Ende eines Schafts für ein medizinisches Instrument werden ein Rohr bereitgestellt und ein im Wesentlichen helikaler Bereich des Rohrs entfernt, um ein Kupplungsglied zu bilden.

Das Rohr weist vor dem Entfernen insbesondere einen kreisringförmigen Querschnitt auf. Der im Wesentlichen helikale Bereich des Rohrs wird insbesondere mittels Laserschneidens entfernt, wobei keine Kräfte auf das Rohr wirken und dieses nicht elastisch oder plastisch verformt wird, wodurch eine hohe Präzision erreichbar ist. Wenn das Kupplungsglied als Kupplungsglied an einem proximalen Ende eines Werkzeugs vorgesehen ist, kann es nach dem Entfernen des im Wesentlichen helikalen Bereichs an das proximale Ende des Werkzeugs gefügt, insbesondere geschweißt, gelötet oder geklebt werden. Alternativ kann bereits vor dem Entfernen des im Wesentlichen helikalen Bereichs das Rohr an das proximale Ende des Werkzeugs gefügt werden.

Wenn das Kupplungsglied für das distale Ende eines Schafts vorgesehen ist, kann das Rohr das Außenrohr des Schafts bilden oder vor oder nach dem Entfernern des im Wesentlichen helikalen Bereichs an ein Außenrohr des Schafts gefügt werden, insbesondere geschweißt, gelötet oder geklebt.

Weitere mögliche Ausführungsformen, Varianten, Eigenschaften, Merkmale, Wirkungen und Vorteile eines medizinischen Instruments entsprechen den oben für ein Werkzeug bzw. einen Schaft beschriebenen.

Bei einem Verfahren, wie es hier beschrieben ist, wird ferner eine Stützhülse an das Kupplungsglied gefügt.

Die Stützhülse kann vor oder nach dem Fügen des Kupplungsglieds an das proximale Ende des Werkzeugs an das Kupplungsglied gefügt werden. Die Stützhülse wird insbesondere mittels Laserschweißens an das Kupplungsglied gefügt. Da die Stützhülse und dessen mechanische Verbindung zum Kupplungslied so vorgesehen und ausgebildet sein können, dass die Stützhülse lediglich einer weiträumigen Übertragung bzw. Ableitung von Kräften ohne lokale mechanische Spannungsspitzen dient, kann sie sehr dünnwandig ausgebildet sein.

Weitere mögliche Ausführungsformen, Varianten, Eigenschaften, Merkmale, Wirkungen und Vorteile eines Verfahrens entsprechen den oben für ein Werkzeug bzw. einen Schaft beschriebenen.

### Kurzbeschreibung der Figuren

Nachfolgend werden Ausführungsformen anhand der beigefügten Figuren näher erläutert. Es zeigen:
- Figur 1: eine schematische Darstellung eines medizinisches Instruments;
- Figur 2: eine schematische axonometrische Darstellung eines Teils eines distalen Endes eines Schafts;
- Figur 3: eine weitere schematische axonometrische Darstellung des distalen Endes aus Figur 2;
- Figur 4: eine schematische Darstellung eines Schnitts durch das distale Ende aus den Figuren 2 und 3;

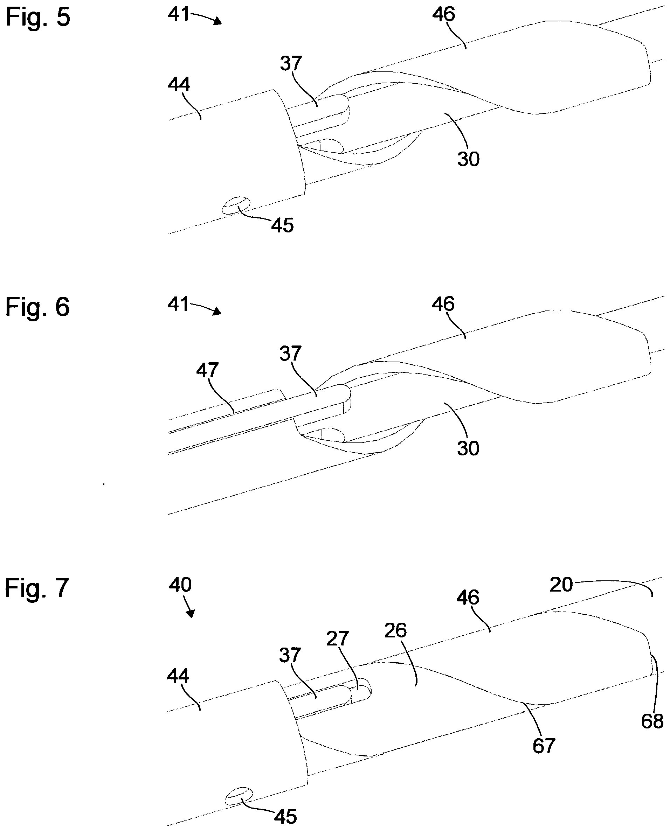

- Figur 5: eine schematische axonometrische Darstellung eines proximalen Endes eines Werkzeugs;
- Figur 6: eine weitere schematische axonometrische Darstellung des proximalen Endes aus Figur 5;
- Figur 7: eine schematische axonometrische Darstellung eines proximalen Endes eines Werkzeugs und eines distalen Endes eines Schafts, die miteinander gekoppelt sind;

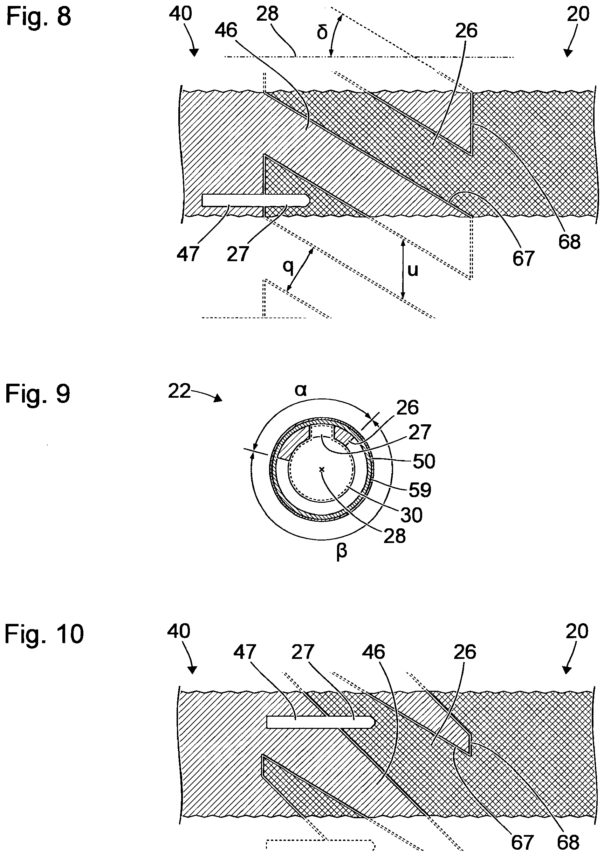

- Figur 8: eine schematische Darstellung einer Abwicklung von Kupplungsgliedern;
- Figur 9: eine schematische Darstellung eines Querschnitts einer Variante eines Kupplungsglieds;
- Figur 10: eine schematische Darstellung einer Abwicklung von Kupplungsgliedern;

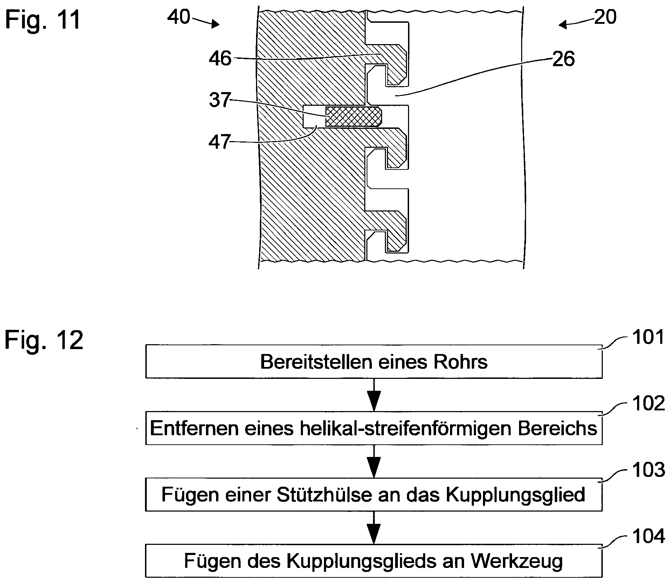

- Figur 11: eine schematische Darstellung einer Abwicklung von Kupplungsgliedern;
- Figur 12: ein schematisches Flussdiagramm.

### Beschreibung der Ausführungsformen

Figur 1 zeigt eine schematische Darstellung eines medizinischen Instruments, insbesondere eines mikroinvasiv-chirurgischen Instruments 10 mit einer Handhabungseinrichtung 15 am proximalen Ende, einem Werkzeug 40 am distalen Ende und einem Schaft 20, der die Handhabungseinrichtung 15 mit dem Werkzeug 40 verbindet. Ein proximales Ende 21 des Schafts 20 ist dauerhaft oder lösbar mit der Handhabungseinrichtung 15 gekoppelt. An der Handhabungseinrichtung 15 ist ein Rad 16 vorgesehen, mittels dessen der Schaft 20 um seine Längsachse 28 rotierbar ist. Die Handhabungseinrichtung 15 umfasst ferner einen schwenkbaren Teil 17, der um eine Schwenkachse 18 senkrecht zur Längsachse 28 des Schafts 20 schwenkbar ist.

Das distale Ende 22 des Schafts ist mit dem proximalen Ende 41 des Werkzeugs 40 lösbar verbunden. Das distale Ende 42 des Werkzeugs 40 bildet gleichzeitig das distale Ende des medizinischen Instruments 10.

Das Werkzeug 40 ist in Figur 1 unten ein zweites Mal vom Schaft 20 getrennt dargestellt. Das Werkzeug 40 umfasst an seinem proximalen Ende 41 ein Kupplungsglied 46 zur lösbaren mechanischen Kopplung mit dem distalen Ende 22 des Schafts 20. Ferner umfasst das Werkzeug 40 ein feststehendes Maulteil und ein schwenkbares Maulteil 48. Alternativ kann das Werkzeug 40 zwei oder mehr schwenkbare Maulteile umfassen. Das Werkzeug 40 kann eine Zange, ein Greifer, eine Schere, ein Nadelhalter oder ein anderes Werkzeug mit einem feststehenden und einem schwenkbaren Maulteil oder mit zwei oder mehr schwenkbaren Maulteilen oder mit einer andersartigen mittels der Übertragungsstange 30 bewegbaren Wirkeinrichtung sein.

Das Werkzeug ist mit einem distalen Ende einer Übertragungsstange 30 zum Übertragen einer Kraft und/oder eines Drehmoments von der Handhabungseinrichtung 15 zum Werkzeug 40 verbunden. Das proximale Ende 31 der Übertragungsstange 30 ist dazu vorgesehen und ausgebildet, mit dem schwenkbaren Teil 17 der Handhabungseinrichtung 15 gekoppelt zu werden. Das distale Ende der Übertragungsstange 30 ist innerhalb des Maulteils 40 angeordnet und deshalb in Figur 1 nicht sichtbar. Das distale Ende der Übertragungsstange 30 ist unmittelbar oder mittelbar (beispielsweise mittels eines Pleuels oder Kniehebels) mit dem schwenkbaren Maulteil 48 des Werkzeugs 40 gekoppelt.

In dem in Figur 1 oben dargestellten zusammengesetzten Zustand des medizinischen Instruments 10 sind Schwenkbewegungen des schwenkbaren Teils 17 der Handhabungseinrichtung 15 und des schwenkbaren Maulteils 48 des Werkzeugs 40 mittels der Übertragungsstange 30 gekoppelt. In durchgezogenen Linien sind das schwenkbare Maulteil 48 des Werkzeugs 40 in einer offenen Position und der schwenkbare Teil 17 der Handhabungseinrichtung 15 in einer korrespondierenden Position dargestellt. In gestrichelten Linien sind das schwenkbare Maulteil 48 in einer überoffenen Position und der schwenkbare Teil 17 der Handhabungseinrichtung 15 in einer korrespondierenden Position dargestellt. Die Bedeutung der überoffenen Position ist unten mit Bezug zu Figur 7 näher beschrieben.

Wenn das schwenkbare Teil 17 von der in Figur 1 in durchgezogener Linie dargestellten Position aus weiter nach distal geschwenkt wird, wird das schwenkbare Maulteil 48 des Werkzeugs 40 von der in durchgezogener Linie dargestellten Position nach unten geschwenkt, wodurch ein Greifen, Klemmen oder Schneiden möglich ist.

Figur 2 zeigt eine schematische axonometrische Darstellung des distalen Endes 22 des Schafts 20 ohne Werkzeug. Der Schaft 20 ist in Figur 2 durch ein Schaftrohr repräsentiert, das sich vom proximalen Ende 21 bis zum distalen Ende 22 (vgl. Figur 1) des Schafts 20 erstreckt. Nachfolgend wird zwischen dem Schaft und dem Schaftrohr nicht unterschieden.

Am distalen Ende 22 ist das Schaftrohr 20 von einer dünnwandigen Hülse 50 mit einem proximalen Rand 51 und einem distalen Rand 52 umgeben. Sowohl das Schaftrohr 20 als auch die Hülse 50 weisen - von nachfolgend beschriebenen Ausnahmen abgesehen - jeweils im Wesentlichen einen kreisringförmigen Querschnitt auf. Am distalen Ende 52 der Stützhülse 50 ist ein nach radial außen ragender Kragen 54 vorgesehen, der den distalen Rand 52 der Stützhülse 50 versteift.

Figur 3 zeigt eine weitere schematische axonometrische Darstellung des distalen Endes 22 des Schafts 20. Die schematische axonometrische Darstellung in Figur 3 entspricht hinsichtlich der Blickrichtung und des dargestellten Ausschnitts der Figur 2. Die Darstellung in Figur 3 unterscheidet sich von der Darstellung in Figur 2 dadurch, dass die Stützhülse 50 entfernt ist.

Das Schaftrohr 20 weist an seinem distalen Ende 22 nicht die Gestalt eines vollständigen Kreiszylinders auf. Vielmehr ist das Schaftrohr 20 nahe seinem distalen Ende 22 zu einem im Wesentlichen helikal-streifenförmigen Kupplungsglied 26 umgestaltet. Das Kupplungsglied 26 weist eine innere Mantelfläche 61 und eine äußere Mantelfläche 62 auf. Die innere und die äußere Mantelfläche 61, 62 sind jeweils Ausschnitte aus Kreiszylindermänteln (im mathematischen Sinne, also als Flächen). Der Rand des Kupplungsglieds 26 umfasst zwei helikale Abschnitte 67 (von denen in Figur 3 nur eines mit dem Bezugszeichen versehen ist) und einen umfänglichen Abschnitt 68.

Das Kupplungsglied 26 weist eine Kerbe 27 auf, deren Funktion unten mit Bezug auf Figur 7 näher beschrieben ist. Die Kerbe 27 erstreckt sich vom distalen Rand des Schaftrohrs 20 bzw. des Kupplungsglieds 26 in Richtung parallel zur Längsachse 28 des Schaftrohrs 20.

Figur 4 zeigt eine schematische Darstellung eines Schnitts durch das distale Ende 22 des Schafts 20 entlang einer Ebene senkrecht zur Längsachse 28. Die Schnittebene liegt nah am distalen Rand unmittelbar proximal des Kragens 54 der Stützhülse 50.

Das Kupplungsglied 26 erstreckt sich in umfänglicher Richtung über einen Winkel α (alpha) von ca. 180°. Das Kupplungsglied 26 wird von der dünnwandigen Stützhülse 50 umschlossen, deren kreisförmiger Querschnitt in Figur 4 erkennbar ist. Die Stützhülse 50 und das Schaftrohr 20 (vgl. Figuren 2 und 3) werden von einem Schrumpfschlauch 59 umschlossen. Der in Figur 2 nicht dargestellte Schrumpfschlauch 59 schließt unmittelbar proximal an den Kragen 54 am distalen Rand 52 der Stützhülse 50 an. Anstelle des Schrumpfschlauchs 59 kann eine andere Ummantelung vorgesehen sein, die insbesondere eine elektrische Isolation des Schafts 20 gegenüber der Umgebung ermöglichen kann.

Im Lumen des Schaftrohrs 20 bzw. des Kupplungsglieds 26 ist in gestrichelter Linie die Kontur einer Übertragungsstange dargestellt, die in den Schaft eingeführt werden kann.

Figur 5 zeigt eine schematische axonometrische Darstellung des proximalen Endes 41 eines Werkzeugs 40, wie es oben anhand der Figur 1 dargestellt ist. Die schematische axonometrische Darstellung der Figur 5 entspricht hinsichtlich der Blickrichtung den Darstellungen der Figuren 2 und 3.

Das Werkzeug 40 weist ein Kupplungsglied 46 mit der Gestalt eines helikal-streifenförmigen Ausschnitts eines Kreiszylindermantels auf. Ferner ist eine Übertragungsstange 30, wie sie oben anhand der Figur 1 dargestellt ist, innerhalb des Kupplungsglieds 26 parallel zu ihrer Längsachse verschiebbar angeordnet. Bei dem in Figur 5 dargestellten Beispiel weist die Übertragungsstange 30 einen kreisförmigen Querschnitt auf, dessen Durchmesser im Sinne einer deutlichen Darstellung und einer deutlichen Unterscheidbarkeit vom Kupplungsglied 46 kleiner ist als der vom Kupplungsglied 46 umschlossene Querschnitt. Abweichend von der Darstellung in Figur 5 kann die Übertragungsstange 30 innen am Kupplungsglied 46 anliegen oder im Wesentlichen anliegen bzw. einen äußeren Querschnitt aufweisen, der nur unwesentlich geringer ist als der innere Querschnitt des Kupplungsglieds 46.

Ferner ist in Figur 4 ein im dargestellten Ausschnitt rohrförmiges Bauteil 44 des Maulteils 40 erkennbar. Das in Figur 4 nicht dargestellte distale Ende des Bauteils 44 umfasst insbesondere ein feststehendes Maulteil und/oder ein oder mehrere schwenkbare Maulteile 48 (vgl. Figur 1). Die Funktion einer Öffnung 45 am Bauteil 44 wird unten mit Bezug auf Figur 6 beschrieben. An der Übertragungsstange 30 ist ein Riegel 37 ausgebildet, dessen Funktion unten mit Bezug auf Figur 7 näher beschrieben ist.

Figur 6 zeigt eine weitere schematische axonometrische Darstellung des proximalen Endes 41 des Werkzeugs 40 aus Figur 5. Die schematische axonometrische Darstellung in Figur 6 entspricht hinsichtlich der Blickrichtung und des dargestellten Ausschnitts der Darstellung in Figur 5.

Die Darstellung in Figur 6 unterscheidet sich von der Darstellung in Figur 5 dadurch, dass das Bauteil 44 des Werkzeugs 40 nicht dargestellt ist. Das distale Ende des Kupplungsglieds 46 ist im Wesentlichen rohrförmig mit einem Längsschlitz bzw. einer Kerbe 47 für den Riegel 37. Die in Figur 5 erkennbare Öffnung 45 im hülsenförmigen Bestandteil des Werkzeugs 40 oder mehrere derartige Öffnungen dienen einer stoffschlüssigen Verbindung des Bauteils 44 mit dem Kupplungsglied 46 durch Laserschweißen oder auf andere Weise.

Figur 7 zeigt eine schematische axonometrische Darstellung einer lösbaren formschlüssigen mechanischen Verbindung des oben anhand der Figuren 1, 5 und 6 dargestellten Werkzeugs 40 mit dem oben anhand der Figuren 1 bis 4 dargestellten distalen Ende des Schafts 20. Die schematische axonometrische Darstellung in Figur 7 entspricht hinsichtlich der Blickrichtung und des dargestellten Ausschnitts den Darstellungen der Figuren 5 und 6.

Das helikal-streifenförmige Kupplungsglied 26 am Schaft 20 und das helikal-streifenförmige Kupplungsglied 46 am Werkzeug 40 greifen ineinander und bilden eine formschlüssige mechanische Verbindung zwischen dem Schaft 20 und dem Werkzeug 40. Dabei ergänzen sich die Kupplungsglieder 26, 46 im Wesentlichen zu einem vollständigen Kreiszylindermantel. Die helikalen Abschnitte 67 und die umfänglichen Abschnitte 68 der Ränder der Kupplungsglieder 26, 46 liegen im Wesentlichen aneinander an oder sind nur jeweils durch einen kleinen Spalt getrennt.

Der Riegel 37 an der Übertragungsstange 30 (vgl. Figuren 5 und 6) greift in die Kerbe 27 am Kupplungsglied 26 ein. Da der Riegel - wie beispielhaft in Figur 6 angedeutet - im Werkzeug 40 nur axial verschiebbar ist, ist die formschlüssige Verbindung der Kupplungsglieder 26, 46 durch den Eingriff des Riegels 37 in die Kerbe 27 verriegelt. Das Werkzeug 40 und insbesondere der Riegel 37 sind so ausgebildet, dass der Riegel 37 nicht mehr in die Kerbe 27 eingreift, wenn das schwenkbare Maulteil 48 des Werkzeugs 40 die in Figur 1 in gestrichelter Linie dargestellte überoffene Position einnimmt. Deshalb kann das Werkzeug 40 in der überoffenen Position des schwenkbaren Maulteils 48 mit dem distalen Ende 22 des Schafts 20 gekoppelt und von diesem gelöst werden.

Figur 8 zeigt eine schematische Darstellung einer Abwicklung der Kupplungsglieder 26, 46 am Schaft 20 und am Werkzeug 40, die oben mit Bezug auf die Figuren 1 bis 7 dargestellt sind. Die Abwicklung entspricht einer Transformation bzw. einer Abbildung eines Kreiszylindermantels in eine Ebene. Die Schnittlinie, entlang derer der Kreiszylinder aufgeschnitten ist, ist gewellt dargestellt und ist ebenso wie die Symmetrieachse des Kreiszylinders horizontal angeordnet.

Die Kupplungsglieder 26, 46 sind zur besseren Unterscheidbarkeit in unterschiedlichen Schraffuren dargestellt. Die Ränder der Kupplungsglieder 26, 46 sind der Einfachheit halber als aus helikalen Abschnitten 67 und umfänglichen Abschnitten 68, die in der Abwicklung jeweils gerade sind, zusammengesetzt dargestellt. Die in den Figuren 3 und 5 bis 7 erkennbaren abgerundeten Übergänge zwischen den helikalen Abschnitten 67 und den umfänglichen Abschnitten 68, die die Kerbwirkung herabsetzen und die Robustheit erhöhen können, sind in Figur 8 der Einfachheit halber nicht dargestellt.

Die helikalen Abschnitte 67 der Ränder der Kupplungsglieder 26, 46 schließen mit der Längsachse 28 (vgl. Figuren 1, 3) einen Winkel δ (delta) ein. Die in umfänglicher Richtung gemessene Breite u jedes helikal-streifenförmigen Kupplungsabschnitts 26, 46 ist die Hälfte des Gesamtumfangs U = π x d (U = pi mal d) des Schaftrohrs 20, wobei d der Durchmesser des Schaftrohrs 20 ist. Der in Figur 4 dargestellte Winkel α (alpha) beträgt (in Radian) α = u/U (alpha = u geteilt durch U). Die senkrecht zu den helikalen Abschnitten 67 der Ränder der Kupplungsglieder 26, 46 gemessene Breite q der Kupplungsglieder 26, 46 beträgt q = u x sin(δ) (q = u mal sin(delta)).

Je größer der Winkel δ (delta) zwischen den helikalen Kanten 67 der Kupplungsglieder 26, 46 und der Längsachse 28 ist, desto kleiner ist die in Richtung senkrecht zu den helikalen Kanten 67 gemessene Breite q. Gleichzeitig nimmt mit zunehmenden δ (delta) jedoch auch die parallel zu den helikalen Kanten 67 gemessene Länge der helikal-streifenförmigen Kupplungsglieder 26, 46 ab. Ein hinsichtlich der mechanischen Steifigkeit der helikal-streifenförmigen Kupplungsglieder 26, 46 vorteilhafter Winkel δ (delta) beträgt ca. 60°.

Figur 9 zeigt eine schematische Darstellung eines Schnitts entlang einer Schnittebene senkrecht zur Längsachse 28 durch ein distales Ende eines Schafts, der in einigen Merkmalen dem oben anhand der Figuren 1 bis 4, 7 und 8 dargestellten Ausführungsbeispiel ähnelt. Die Darstellung in Figur 9 entspricht hinsichtlich der Lage der dargestellten Schnittebene unmittelbar proximal eines Kragens 54 am distalen Ende 52 einer Stützhülse 50 der Darstellung in Figur 4.

Das in Figur 9 dargestellte Ausführungsbeispiel unterscheidet sich von dem oben anhand der Figuren 1 bis 8 dargestellten Ausführungsbeispiel dadurch, dass die beiden korrespondierenden Kupplungsglieder 26, 46 unterschiedlich breit sind. Das Kupplungsglied 26 am distalen Ende des Schafts weist in umfänglicher Richtung eine Breite α (alpha) auf, die ca. 120° beträgt. Das in Figur 9 nicht dargestellte Kupplungsglied am distalen Ende des Schafts erstreckt sich in umfänglicher Richtung über einen Winkel β (beta) von ca. 240°. Da die Steifigkeit des Kupplungsglieds 26 am distalen Ende des Schafts 20 durch die Stützhülse 50 erhöht wird, können mit unterschiedlichen Breiten der Kupplungsglieder näherungsweise gleiche mechanische Steifigkeiten erzielt werden.

Die Figuren 10 und 11 zeigen schematische Darstellungen zweier weiterer Ausführungsbeispiele einer mechanischen Kopplung eines Werkzeugs 20 mit einem Schaft 40. Die Darstellungen in den Figuren 10 und 11 zeigen entsprechend der Darstellung in Figur 8 Abwicklung von Kupplungsgliedern 26, 46 am Schaft 20 und am Werkzeug 40 in die Zeichenebene. Die Ausführungsbeispiele der Figuren 10 und 11 ähneln jeweils dem Ausführungsbeispiel der Figuren 1 bis 8 in einigen Merkmalen, die nachfolgend nicht noch einmal beschrieben sind.

Bei dem in Figur 10 dargestellten Ausführungsbeispiel weisen die Kupplungsglieder 26, 46 an Schaft 20 und Werkzeug 40 jeweils die Gestalt eines im Wesentlichen helikal-keilförmigen Ausschnitts aus einem Kreiszylindermantel auf. Die beiden helikalen Abschnitte 67 der Ränder eines Kupplungsglieds 26, 46 haben unterschiedliche Steigungen bzw. schließen mit der Längsachse 28 (vgl. Figuren 1, 3, 8) unterschiedliche Winkel ein. Deshalb nehmen die in Richtung des Umfangs des Kreiszylindermantels bzw. senkrecht zur Längsachse 28 gemessene Breite des Kupplungsglieds 26 am Schaft 20 nach distal (in Figur 10 links) ab und die Breite des Kupplungsglieds 46 am Werkzeug 40 nach proximal (in Figur 10 rechts) ab.

Abweichend von der helikal-keilförmigen Gestalt sind am Kupplungsglied 26 am Schaft 20 eine Kerbe 27 und am Kupplungsglied 46 am Werkzeug 40 eine Kerbe 47 vorgesehen. Ähnlich wie bei dem anhand der Figuren 1 bis 7 dargestellten Ausführungsbeispiel ist ein in Figur 10 nicht dargestellter Riegel axial bzw. parallel zur Längsachse 28 in der Kerbe 47 verschiebbar. Wenn der Riegel gleichzeitig in die Kerbe 47 im Werkzeug 40 und in die Kerbe 27 im Schaft 20 eingreift, verriegelt er die mechanische Kopplung zwischen Werkzeug 40 und Schaft 20.

Bei dem in Figur 11 dargestellten Ausführungsbeispiel weisen die Kupplungsglieder 26, 46 an Schaft 20 und Werkzeug 40 jeweils eine im Wesentlichen hakenförmige bzw. L-förmige Gestalt auf. Sowohl der Schaft 20 als auch das Werkzeug 40 weisen jeweils über den Umgang gleichmäßig verteilt hakenförmige Kupplungsglieder 26, 46 auf. Die Anzahl der hakenförmgien Kupplungsglieder 26 am Schaft entspricht der Anzahl der Kupplungsglieder 46 am Werkzeug. Bei dem dargestellten Beispiel weisen der Schaft 20 und das Werkzeug 40 jeweils drei Kupplungsglieder 26, 46 auf.

Jedes Kupplungsglied 26 am Schaft 20 weist einen axialen Abschnitt, der sich parallel zur Längsachse 28 (vgl. Figuren 1, 3, 8) erstreckt, und an dessen distalem Ende einen umfänglichen Abschnitt, der sich senkrecht zur Längsachse 28 erstreckt, auf. Jedes Kupplungsglied 46 am Werkzeug 40 weist einen axialen Abschnitt, der sich parallel zur Längsachse 28 erstreckt, und an dessen proximalem Ende einen umfänglichen Abschnitt, der sich senkrecht zur Längsachse 28 erstreckt, auf.

Die Kupplungsglieder sind insbesondere hinsichtlich ihrer Abmessungen und ihrer Abstände so ausgebildet, dass durch eine axiale und eine anschließende rotatorische Relativbewegung von Werkzeug 40 und Schaft 20 die in Figur 11 dargestellte mechanisch gekoppelte Situation herstellbar ist. Dabei liegen die umfänglichen Abschnitte der Kupplungsglieder 46 am Werkzeug 40 teilweise proximal der umfänglichen Abschnitte der Kupplungsglieder 26 am Schaft 20. Der Riegel 37 kann die mechanische Kopplung von Werkzeug 40 und Schaft 20 verriegeln. Durch eine umgekehrte rotatorische und eine anschließende axiale Relativbewegung kann die in Figur 11 dargestellte mechanisch gekoppelte Situation wieder aufgelöst werden.

Bei den oben anhand der Figuren 1 bis 11 dargestellten Ausführungsbeispielen ist da Kupplungsglied 26, 46 an einem proximalen Ende 41 eines Werkzeugs 40 oder an einem distalen Ende 22 eines Schafts 20 angeordnet. Bei dieser Verwendung des Kupplungsglieds 26, 46 kann von Vorteil sein, dass das Kupplungsglied 26, 46 in besonderem Maße miniaturisierbar sein kann. Dies kann besonders geringe Querschnitte des Schafts und des Werkzeugs ermöglichen. Jedoch sind ähnliche Kupplungsglieder alternativ am proximalen Ende 21 eines Schafts 20 (zur lösbaren mechanischen Kopplung mit einem distalen Ende eines weiteren Schafts oder Schaftabschnitts oder mit einem distalen Ende einer Handhabungseinrichtung) oder am distalen Ende einer Handhabungseinrichtung 15 (zur lösbaren mechanischen Kopplung mit einem proximalen Ende 21 eines Schafts 20) verwendbar.

Figur 12 zeigt ein schematisches Flussdiagramm eines Verfahrens zum Herstellen eines Kupplungsglieds für oder an einem proximalen Ende eines Werkzeugs oder für oder an einem distalen Ende eines Schafts oder für oder an einem proximalen Ende eines Schafts oder für oder an einem distalen Ende einer Handhabungseinrichtung für ein medizinisches Instrument. Obwohl das Verfahren auch verwendbar ist, um Kupplungsglieder herzustellen, die sich von den oben anhand der Figuren 1 bis 9 dargestellten unterscheiden, werden nachfolgend beispielhaft Bezugszeichen aus den Figuren 1 bis 9 verwendet, um die Verständlichkeit zu verbessern.

Bei einem ersten Schritt 101 wird ein Rohr mit einem kreisringförmigen Querschnitt bereitgestellt. Wenn ein Kupplungsglied an einem proximalen Ende 21 oder an einem distalen Ende 22 eines Schafts 20 hergestellt werden soll, kann das Rohr das den Schaft 20 oder dessen wesentlichen Bestandteil bildende Schaftrohr sein.

Bei einem zweiten Schritt 102 wird ein Bereich des Rohrs entfernt, um das Kupplungsglied 26, 46 zu bilden. Insbesondere wird ein helikale-keilförmiger oder helikal-streifenförmiger Bereich entfernt, wobei das Kupplungsglied 26, 46 ebenfalls in helikal-keilförmiger oder helikal-streifenförmiger Gestalt entsteht. Optional kann ferner eine Kerbe 27 für eine Verriegelung mittels eines in die Kerbe 27 eingreifenden Riegels 37 an dem Kupplungsglied erzeugt werden.

Bei einem optionalen dritten Schritt 103 wird eine Stützhülse 50 an das Kupplungsglied 26, 46 gefügt, insbesondere geschweißt, gelötet oder geklebt.

Wenn das Rohr, an dem das Kupplungsglied 26, 46 beim zweiten Schritt 102 gebildet wurde, nicht das Schaftrohr eines Schafts 20 ist, kann bei einem vierten Schritt 104 das Kupplungsglied 26, 46 (ggf. zusammen mit der Stützhülse 50) an das proximale Ende des Werkzeugs bzw. an das proximale Ende 21 oder an das distale Ende des Schafts 20 gefügt werden. Alternativ kann der vierte Schritt 104 bereits vor dem zweiten Schritt 102 ausgeführt werden.

### Bezugszeichen

- 10: medizinisches Instrument
- 15: Handhabungseinrichtung am proximalen Ende des medizinischen Instruments 10
- 16: Rad an der Handhabungseinrichtung 15
- 17: schwenkbarer Teil der Handhabungseinrichtung 15
- 18: Schwenkachse des schwenkbaren Teils 17
- 20: Schaft des medizinischen Instruments 10
- 21: proximales Ende des Schafts 20
- 22: distales Ende des Schafts 20
- 26: Kupplungsglied am distalen Ende 41 des Schafts 20
- 27: Kerbe am Kupplungsglied 26
- 28: Längsachse des Schafts 20
- 30: Übertragungsstange
- 31: proximales Ende der Übertragungsstange 30
- 37: Riegel am distalen Ende der Übertragungsstange 30
- 40: Werkzeug
- 41: proximales Ende des Werkzeugs 40
- 42: distales Ende des Werkzeugs 40
- 44: Bauteil des Werkzeugs 40
- 45: Öffnung für Schweißverbindung
- 46: Kupplungsglied am proximalen Ende 41 des Werkzeugs 40
- 47: Kerbe am Kupplungsglied 46
- 48: schwenkbares Maulteil des Werkzeugs 40
- 50: Stützhülse am Kupplungsglied 26
- 51: proximaler Rand der Stützhülse 50
- 52: distaler Rand der Stützhülse 50
- 54: Kragen am distalen Rand 52 der Stützhülse 50
- 59: Schrumpfschlauch
- 61: innere Mantelfläche
- 62: äußere Mantelfläche
- 67: helikaler Abschnitt des Rands des Kupplungsglieds 26
- 68: umfänglicher Abschnitt des Rands des Kupplungsglieds 26
- 101: erster Schritt (Bereitstellen eines Rohrs)
- 102: zweiter Schritt (Entfernen eines helikal-streifenförmigen Bereichs des Rohrs)
- 103: dritter Schritt (Fügen einer Stützhülse an das Kupplungsglied)
- 104: vierter Schritt (Fügen des Kupplungsglieds an das proximale Ende eines Werkzeugs)
- α: (alpha) Winkel, über den sich das Kupplungsglied 26 in umfänglicher Richtung erstreckt
- β: (beta) Winkel, über den sich das Kupplungsglied 46 in umfänglicher Richtung erstreckt
- δ: (delta) Winkel zwischen helikalem Abschnitt 67 und Längsachse 28
- q: Breite des Kupplungsglieds 26 gemessen senkrecht zum helikalen Abschnitt 67
- u: Breite des Kupplungsglieds 26 gemessen in umfänglicher Richtung

## Patentansprüche

1. Zerstörungsfrei zerlegbares medizinisches Instrument (10) mit: einem Schaft (20),
einem Werkzeug (40),
einer Kupplung an dem Schaft (20) mit
einem Kupplungsglied (26) am distalen Ende des Schafts (20) zur lösbaren mechanischen Kopplung mit einem korrespondierenden Kupplungsglied (46) am proximalen Ende (41) eines Werkzeugs (40), **dadurch gekennzeichnet, dass** das Kupplungsglied (26) am distalen Ende des Schafts (20) die Gestalt eines im Wesentlichen helikalen Ausschnitts eines Kreiszylindermantels aufweist, wobei das Kupplungsglied (26) am distalen Ende des Schafts (20) eine Kerbe (27) aufweist,
die sich vom distalen Rand des Schaftrohrs (20) bzw. des Kupplungsglieds (26) am distalen Ende des Schafts (20) in Richtung parallel zur Längsachse (28) des Schaftrohrs (20) erstreckt,
wobei das Werkzeug (40) einen Riegel (37) umfasst, der relativ zum Kupplungsglied (26) axial verschiebbar ist und in die Kerbe (27) am Kupplungsglied (26) eingreift.

2. Medizinisches Instrument nach Anspruch 1, **dadurch gekennzeichnet, dass** das Kupplungsglied (26) am distalen Ende des Schafts (20) und das Kupplungsglied (46) am proximalen Ende (41) des Werkzeugs (40) die Gestalt eines helikal-streifenförmigen Ausschnitts eines Kreiszylindermantels, dessen Breite nach proximal kontinuierlich oder diskontinuierlich abnimmt, aufweist.

3. Medizinisches Instrument nach einem der vorhergehenden Ansprüche, ferner mit:
einer Stützhülse (50), die an dem Kupplungsglied (26, 46) anliegt und mit dem Kupplungsglied (26, 46) gefügt ist.

4. Medizinisches Instrument nach einem der vorhergehenden Ansprüche, bei dem der helikal-streifenförmige Ausschnitt des Kreiszylindermantels in umfänglicher Richtung eine Breite entsprechend dem halben Umfang des Kreiszylindermantels aufweist.

## Claims

1. Medical instrument (10) able to be taken apart in a non-destructive manner, with: a shaft (20),
a tool (40),
a coupling on the shaft (20) with
a coupling member (26) at the distal end of the shaft (20) for releasable mechanical coupling to a corresponding coupling member (46) at the proximal end (41) of a tool (40),
**characterized in that**
the coupling member (26) at the distal end of the shaft (20) has the form of a substantially helical cutout of a circular cylindrical barrel,
wherein the coupling member (26) at the distal end of the shaft (20) has a notch (27),
which extends from the distal edge of the shaft tube (20), or of the coupling member (26) at the distal end of the shaft (20), in a direction parallel to the longitudinal axis (28) of the shaft tube (20),
wherein the tool (40) comprises a bolt (37), which is axially displaceable relative to the coupling member (26) and engages in the notch (27) at the coupling member (26).

2. Medical instrument according to Claim 1, **characterized in that** the coupling member (26) at the distal end of the shaft (20) and the coupling member (46) at the proximal end (41) of the tool (40) have the form of a helical-strip-shaped cutout of a circular cylindrical barrel, of which the width decreases continuously or discontinuously in the proximal direction.

3. Medical instrument according to one of the preceding claims, moreover with:
a support sleeve (50), which bears on the coupling member (26, 46) and is joined to the coupling member (26, 46).

4. Medical instrument according to one of the preceding claims, in which the helical-strip-shaped cutout of the circular cylindrical barrel has in the circumferential direction a width corresponding to half the circumference of the circular cylindrical barrel.

## Revendications

1. Instrument (10) médical démontable de façon non destructive, avec : un manche (20),
un outil (40),
un accouplement sur le manche (20) avec
un organe d'accouplement (26) à l'extrémité distale du manche (20) pour le couplage mécanique détachable avec un organe d'accouplement (46) correspondant à l'extrémité proximale (41) d'un outil (40),
**caractérisé en ce que**
l'organe d'accouplement (26) à l'extrémité distale du manche (20) présente la forme d'un secteur essentiellement hélicoïdal d'une enveloppe de cylindre circulaire,
l'organe d'accouplement (26) présentant à l'extrémité distale du manche (20) une entaille (27),
qui s'étend du bord distal du tube de manche (20) ou de l'organe d'accouplement (26) à l'extrémité distale du manche (20) dans une direction parallèle à l'axe longitudinal (28) du tube de manche (20),
l'outil (40) présentant une targette (37) qui peut être déplacée axialement relativement à l'organe d'accouplement (26) et qui engrène dans l'entaille (27) sur l'organe d'accouplement (26).

2. Instrument médical selon la revendication 1,
**caractérisé en ce que**
l'organe d'accouplement (26) à l'extrémité distale du manche (20) et l'organe d'accouplement (46) à l'extrémité proximale (41) de l'outil (40) présentent la forme d'un secteur en forme de bande hélicoïdale d'une enveloppe de cylindre circulaire dont la largeur diminue vers la partie proximale de façon continue ou discontinue.

3. Instrument médical selon l'une des revendications précédentes, en outre avec :
une douille d'appui (50) qui est adjacente à l'organe d'accouplement (26, 46) et qui est assemblée avec l'organe d'accouplement (26, 46).

4. Instrument médical selon l'une des revendications précédentes, dans lequel le secteur en forme de bande hélicoïdale de l'enveloppe de cylindre circulaire présente, dans la direction circonférentielle, une largeur correspondant à la demi-circonférence de l'enveloppe de cylindre circulaire.
